# EUROPEAN PATENT APPLICATION

(11) **EP 4 163 266 A1**
(43) Date of publication of application: **12.04.2023**
(21) Application number: 21818173.3
(22) Date of filing: 28.05.2021
(51) Int. Cl.: C07C 1/12, C07C 9/04, F27D 17/00, C07B 61/00, C04B 7/44, C10L 3/08, B01D 53/14, B01D 53/62, C01B 32/50

(54) **METHOD FOR SEPARATING AND RECOVERING CO2 IN CEMENT PRODUCTION EXHAUST GAS, AND CO2 SEPARATION AND RECOVERY DEVICE**

(30) Priority: 04.06.2020 JP 2020097644; 04.06.2020 JP 2020097645; 11.06.2020 JP 2020101457
(71) Applicant: Mitsubishi Materials Corporation, Tokyo 100-8117 (JP)
(72) Inventor: TAKAYAMA, Yoshinori, Naka-shi, Ibaraki 311-0102 (JP); KAWASAKI, Hajime, Iwaki-shi, Fukushima 971-8101 (JP); KOMA, Takuma, Iwaki-shi, Fukushima 971-8101 (JP); KOMATSU, Takuya, Naka-shi, Ibaraki 311-0102 (JP)
(74) Representative: Gille Hrabal Partnerschaftsgesellschaft mbB Patentanwälte
(86) International application number: PCT/JP2021/020405
(87) International publication number: WO 2021/246317

(57) **Abstract**

A CO₂ separation/recover method in cement production exhaust gas has a step of harmful component removal that removes an acidic component and a harmful component from exhaust gas discharged from a cement production facility; and a step of CO₂ separation and recover that separates and recovers CO₂ by bringing the exhaust gas from which the acidic component and the harmful component are removed into contact with a CO₂ absorption material, so that the acidic component and the harmful component are removed before separating and recovering CO₂, resulting in deterioration of the absorbing ability of the CO₂ absorption material being suppressed; and the cement production exhaust gas can be appropriately disposed.

## Description

### BACKGROUND OF THE INVENTION

### Technical Field

The present invention relates to a CO₂ separation and recover method and a CO₂ separation and recovery device in cement production exhaust gas.

Priority is claimed on Japanese Patent Application Nos. 2020-97644 and 2020-97645, filed June 4, 2020, and Japanese Patent Application No. 2020-101457, filed June 11, 2020, which are incorporated herein by reference.

### Background Art

In various combustion facilities such as thermal power generation or the like, in order to reduce greenhouse gas, it is made effort to reduce CO₂ that is generated and exhausted by combustion. Particularly, since most of energy which is necessary for a social activity is obtained by fossil fuel such as coal, petroleum, natural gas and the like and an amount of CO₂ generated from the foil fuel is stupendous, it is effective to reduce CO₂ originated from the energy for suppressing global warming.

As a technique for reducing CO₂ in combustion exhaust gas, conventionally, for example, the methanation method described in Patent Literature 1 is known. That is a method to obtain methane by separating carbon dioxide contained in the combustion exhaust gas to react with hydrogen. In this methanation method, a step of absorbing carbon dioxide in the combustion exhaust gas by bringing the combustion exhaust gas into contact with a carbon dioxide absorber, a step of taking out gas mainly having carbon dioxide by heating the carbon dioxide absorber that absorbed carbon dioxide, a step of removing sulfur compound in the gas by adding a first amount of hydrogen to the gas mainly having carbon dioxide and then throwing into a desulfurizer which is filled with desulfurizing agent, and a step of converting into methane by adding a second amount of hydrogen to the gas after the step of removing sulfur to convert by methanation reaction through methanation catalyst.

In this methanation method, carbon dioxide to which hydrogen is added passes through the desulfurizer that is filled with desulfurizing agent to remove sulfur compound in the gas.

### Citation List

[Patent Literature 1] Japanese Unexamined Patent Application, First Publication No. 2019-172595

### SUMMARY OF INVENTION

### Technical Problem

However, since the exhaust gas (cement production exhaust gas) of the cement facility includes a large amount of oxides other than the sulfur compound, for example, even if the desulfurizer as described in Patent Literature 1 is used, it is not possible to appropriately dispose the cement production exhaust gas.

The present invention is achieved in consideration of the above circumstances, and has an object to provide a CO₂ separation and recover method and a CO₂ separation and recover device in cement production exhaust gas which can dispose the cement production exhaust gas appropriately.

### Solution to Problem

A CO₂ separation and recover method in cement production exhaust gas according to the present invention removes an acidic component and a harmful component, before separating and recovering CO₂ by bringing exhaust gas from a cement production facility into contact with a CO₂ absorption material.

Here, the acidic components such as SOx, NOx, halogen and the like and the harmful components such as H₂O, dusts and the like may influence the CO₂ absorption material to deteriorate CO₂ absorbing ability. Moreover, there is a possibility that dusts (mainly powder of cement raw material) included in the cement production exhaust gas adhere in pipes of the device and generates scales to increase pressure loss of the pipes, and decrease a gas amount which can be disposed in a CO₂ separation and recover device.

In the present invention, by removing the acidic component and the harmful component before separating and recovering the exhaust gas from the cement production facility, it is possible to suppress the deterioration of the absorbing ability of the CO₂ absorption material. Accordingly, CO₂ can be efficiently recovered from the cement production exhaust gas.

A CO₂ separation and recover device in cement production exhaust gas of the present invention includes a cement production facility that is provided with a harmful component removal unit that removes an acidic component and a harmful component from exhaust gas from the cement production facility, and a CO₂ separation and recover unit that separates and recovers CO₂ by bringing the exhaust from which the acidic component and the harmful component are removed into contact with a CO₂ absorption material.

### Advantageous Effects of Invention

According to the present invention, it is possible to suppress deterioration of absorbing ability of CO₂ absorption material by removing an acidic component and a harmful component appropriately from CO₂ in exhaust gas of a cement production facility.

### BRIEF DESCRIPTION OF DRAWINGS

[FIG. 1] It is a flowchart showing a process of a CO₂ utilizing method in cement production exhaust gas according to one embodiment of the present invention.
[FIG. 2] It is a view simply showing the CO₂ utilizing system in the cement production exhaust gas of the above-described embodiment.
[FIG. 3] It is a block diagram showing a schematic composition of a methanation device configuring the CO₂ separation and recover device in the cement production exhaust gas of the above-described embodiment.

### DESCRIPTION OF EMBODIMENTS

An embodiment of a CO₂ separation and recover method in cement production exhaust gas and a CO₂ separation and recover device in cement production exhaust gas of the present invention will be explained below referring drawings.

This embodiment is an example of removing an acidic component and a harmful component appropriately from CO₂ in the cement production exhaust gas, then generating methane to utilize the methane as a part of fossil fuel or an entire alternative fuel to the cement production facility.

### [Composition of CO₂ Utilizing System]

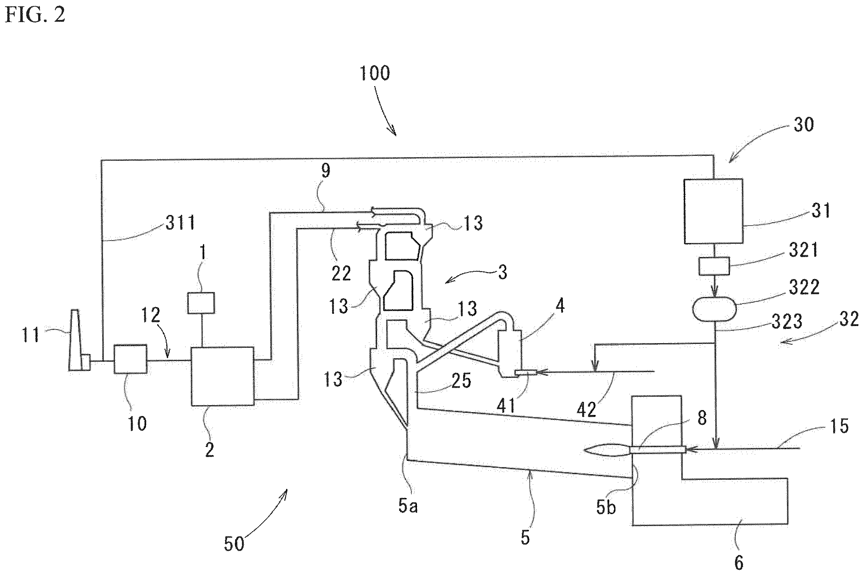

A CO₂ utilizing system 100 is provided with a cement production facility 50 and an exhaust gas treatment facility 30 used by connected to the cement production facility 50 as shown in FIG. 2. In this embodiment, the exhaust gas treatment facility 30 adds hydrogen to CO₂ to the exhaust gas from the cement production facility 50 or CO₂ that is separated and recovered from the exhaust gas to generate methane, and supplies the generated methane as an alternative fuel to a part or entire of the fossil fuel to the cement production facility 50.

### [Composition of Cement Production Facility]

The cement production facility 50 is provided with, as the whole is shown in FIG. 2, a raw material storehouse 1 individually storing lime stone, clay, silica stone, iron material and the like as cement materials, a material mill and a dryer (hereinafter "material mill/dryer") 2 milling and drying these cement materials, a preheater 3 preheating the powdery cement materials supplied via a material supply pipe 22 and obtained by the material mill, a calcination furnace 4 calcining the cement materials preheated by the preheater 3, a cement burning kiln 5 burning the cement materials that are calcined, and a cooler 6 and the like to cool cement clinker after burned in the cement burning kiln 5.

The cement burning kiln 5 is a lateral cylindrical rotary kiln which is slightly inclined, rotated around an axis to send the cement materials supplied in a kiln tail part 5a from the preheater 3 to a kiln front part 5b, and heats and burns to generate cement clinker at about 1450°C by a burner 8 at the kiln front part 5b while sending. The generated cement clinker is sent out from the kiln front part 5b to the cooler 6. To the burner 8, a fuel supply line 15 supplying fuel containing fossil fuel such as coal, petroleum and the like is connected. Other than the fuel supply line 15, in order to enlarge the heat energy, a supplying system (not illustrated) of an alternative heat source such as waste plastic, waste tires and the like is also provided. The cement clinker is cooled in the cooler 6 to a prescribed temperature, then sent to a finishing step.

The preheater 3 is configured so that a plurality (four in an example shown in FIG. 2) of cyclones 13 that flow the exhaust gas occurs in the cement burning kiln 5 are vertically connected as shown in FIG. 2. Between the lowermost cyclone 13 and the next cyclone 13, the calcination furnace 4 is connected. The cement material calcined by the combustion gas of the calcination furnace 4 is supplied to the kiln tail part 5a of the cement burning kiln 5 from the lowermost cyclone 13.

The calcination furnace 4 has a burner 41 therein, and burns fuel such as coal or the like supplied from a fuel supply line 42, thereby calcining the cement material sent from the upper cyclone 13, and supplying the calcined cement material to the lowermost cyclone 13 through a riser duct 25 together with the exhaust gas generated by the calcination. The cement material is supplied from the lowermost cyclone 13 to the kiln tail part 5a of the cement burning kiln 5. The riser duct 25 sends the exhaust gas from the kiln tail part 5a of the cement burning kiln 5 to the lowermost cyclone 13, and the exhaust gas occurs in the calcination furnace 4 is also supplied to the cyclone 13 through the riser duct 25. Therefore, the exhaust gas of the cement burning kiln 5 and the exhaust gas from the calcination furnace 4 go together thorough the preheater 3 from the lower side to the upper side and then are introduced into the material mill/dryer 2 passing through an exhaust pipe 9.

The material mill/dryer 2 carries out pulverization and drying of the cement material simultaneously by introducing the exhaust gas from the calcination furnace 4 and the cement burning kiln 5. To the material mill/dryer 2, an exhaust gas treatment line 12 having a dust collector 10, a chimney 11 and the like is connected.

### [Configuration of Exhaust Gas Treatment Facility]

The exhaust gas treatment facility 30 is provided with an exhaust gas collection line 311 collecting the exhaust gas occurred in the cement burning kiln 5 and the calcination furnace 4 before discharged from the chimney 11 a methanation device 31 separating and recovering CO₂ from the exhaust gas sent from the exhaust gas collection line 311 and adding hydrogen to the separated and recovered CO₂ to generate methane, and a methane supply device 32 supplying the generated methane to the cement production facility 50.

The exhaust gas collection line 311 is connected between the dust collector 10 and the chimney 11 in the exhaust gas treatment line 12 of the cement production facility 50, and collects a part of the exhaust gas generated during cement burning. Since it is the exhaust gas is generated by burning of cement, an exhaust gas due to combustion of fuel such as coal is partially included, but it contains a large amount of exhaust gas originated from lime stone.

### (Configuration of Methanation Device)

The methanation device 31 is provided with a CO₂ separation/recover device 310 that separates and recovers CO₂ from the exhaust gas, a hydrogen mixing unit 316 that supplies and mixes hydrogen to CO₂ separated and recovered by the CO₂ separation/recover device 310 and a methane production part 317 that generates methane from CO₂ in which hydrogen is mixed.

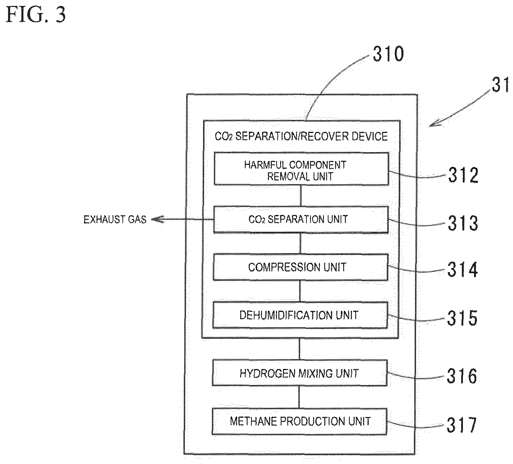

As shown in FIG. 3, the CO₂ separation/recover device 310 is provided with, a harmful component removal unit 312 that removes harmful components such as SOx, NOx and the like from the exhaust gas collected in the exhaust gas collection line 311, a CO₂ separation/recover unit 313 that separates and recovers CO₂ from the exhaust gas from which the harmful components are removed, a compression unit 314 that compresses the recovered CO₂, and a dehumidification unit 315 that removes moisture from the compressed CO₂.

Since the exhaust gas sent from the exhaust gas collection line 311 is combustion exhaust gas of fossil fuel such as coal, petroleum coke, heavy oil or the like, waste plastic, waste tires and the like, CO₂ is contained at about 20% or more for example, and the other gas than CO₂, an acidic component, and a harmful component are contained. Therefore, the harmful component removal unit 312 removes the acidic component (for example, acidification gas such as nitrogen oxides [NOx] or sulfur oxides [SOx]) and the harmful component (H₂O, dusts and the like) from the exhaust gas, and is provided with a scrubber that is filled with an aqueous NaOH and the like, a dehumidifier, an electrostatic precipitator, and the like. By removing the acidic component and the harmful component, halogen is also removed with NOx, an absorption material (CO₂ absorption material) of amine compound used for the next separation and recover of CO₂ is prevented from deteriorating, and the deterioration of the absorbing ability is suppressed.

As a method of desulfurization (SOx removal method), a wet lime-gypsum method, magnesium hydroxide method, and a soda absorption method are known. These are methods of absorbing SOx in alkaline solution; and there is a coalash utilizing method as a dry method. As methods of treating desulfurization (removal of SOx) and denitration (removal of NOx) simultaneously, there are an activated carbon method that is a dry method, and an electron beam method. As the denitration method, there are a catalytic reduction method (SCR method) and a non-catalytic reduction process as dry methods; and an oxidation absorbing method, an oxidation reduction method, and an equimolar absorption method as wet methods.

Since a suspension preheater unit works as a desulfurizing unit, in the exhaust gas from the cement production facility, there are many cases in which SOX is several-ten ppm, generally.

Here, it seems that by an influence of impurities on the absorption material for CO2 separation and recover, affinity between amine compound and impurity compound does not largely differ in either a chemical absorption method and a physical absorption method. In recover of CO₂ by the CO₂ separation/recover unit 313, SOx in the exhaust gas is combined with the amine compound in the absorption material and impedes the CO₂ absorbing ability of the amine compound, so that the absorption rate is largely decreased as time passes. As described above, the amine compound has strong basicity, and other than SOx, NOx and halogen such as Cl, F and the like tend to be absorbed by the CO₂ absorption material.

A Ni catalyst which is broadly used as a catalyst for methanation deteriorates a yield of the methanation under the sulfur compound mixed in H₂ since the sulfur compound reacts on a surface of the Ni catalyst and covers the surface, so that it is necessary to be removed in the harmful component removal unit 312. Similarly, NOx and halogen such as Cl, F and the like may be adhered to the surface of the Ni catalyst and deteriorate the yield of the methanation, so that it is necessary to be removed in the harmful component removal unit 312.

In the present embodiment, any of the above-described methods may be used for desulfurization (removal of SOx) and denitration (removal of NOx). For example, in the present embodiment, any of the above-described desulfurization methods and any of the above-described denitration methods are used to remove the acidification gas such as the nitrogen oxides (NOx), the sulfur oxides (SOx), and the like.

The CO₂ separation/recover unit 313 is made of a standard CO₂ recover device, and is provided with a CO₂ absorption material (a liquid absorption material in which an amine compound is dissolved in water, a solid absorption material in which an amine compound is supported on a porous material, and the like) that absorbs CO₂ in the exhaust gas when the exhaust gas in which the harmful matters are removed comes into contact therewith. Then, by heating the CO₂ absorption material that has absorbed CO₂ and the like, CO₂ is taken out from the CO₂ absorption material and recovered. The CO₂ separation/recover unit 313 discharges the exhaust gas after removing CO₂ to the exterior. The compression unit 314 compresses the recovered CO₂ by applying a pressure of 0.1 MPa or more, preferably 0.5 to 1.0 MPa. The dehumidification unit 315 removes moisture contained in CO₂ by cooling the compressed CO₂. This dehumidification is carried out in order to remove moisture before methanation since the moisture affects the oxidation of a Ni-based catalyst in the methanation device.

The hydrogen mixing unit 316 supplies hydrogen (for example, hydrogen gas) to the dehumidified CO₂, mixes, and compresses it. The hydrogen produced by artificial light synthesis using renewable energy, decomposition of water, or the like can be utilized. The amount of hydrogen added by the hydrogen mixing unit 316 is appropriately set so that methane can be easily produced from CO₂ in which hydrogen is mixed.

The methane production unit 317 generates methane from CO₂ in which hydrogen is mixed. The methane production unit 317 is composed of a general methane production apparatus, provided with a plurality of reactors (not illustrated) filled with a catalyst exhibiting activity in methanation, and produces methane by supplying and reacting CO₂ in which hydrogen is mixed with these reactors. For example, Ni, Pt, Pd, and Cu are used as a hydrogenation catalyst; in the methanation catalyst, particularly, Ni and Ni alloy on which Al₂O₃, Cr₂O₃, SiO₂, MgAl₂O₄, TiO₂, ZrO₂ or the like are supported.

Conditions for a general methanation reaction (CO₂ + 4H₂ → 2H₂O) have a temperature of 200°C to 700°C, preferably 200°C to 350°C, and a pressure of 0.1 to 3 MPa, and it is reacted in multiple stage in order to improve the reaction yield of methane.

### (Configuration of Methane Supply Device)

As shown in FIG. 2, the methane supply device 32 is provided with a tank 322 compressing methane produced by the methanation device 31 by a pump 321 and storing it, and a methane supply line 323 that is connected to the tank 322 and sends methane to the burner 8 of the kiln front part 5b and the burner 41 of the calcination furnace 4 respectively. The methane supply line 321 is connected to the fuel supply line 15 supplying fuel such as coal, petroleum or the like to the burner 8 of the cement burning kiln 5, and the fuel supply line 42 supplying fuel such as coal or the like to the burner 41 of the calcination furnace 4. As a result, methane is supplied to the burners 8 and 41 together with the fuel.

### [CO₂ Utilizing Method]

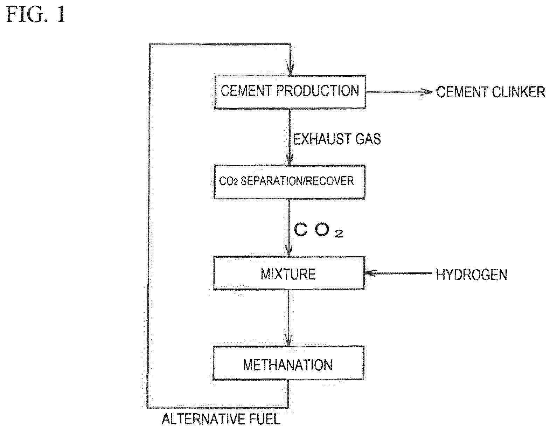

A method of reducing CO₂ in the exhaust gas of the cement production facility 50 and effectively utilizing it using the above-described CO₂ utilizing system will be explained with the flowchart shown in FIG. 1.

In the cement production facility 50, the powdery cement material obtained by milling and drying lime stone, clay, silica stone, iron material and the like as the cement material is preheated; the preheated cement material is subjected to the calcination and then burned, and cooled, so that the cement clinker is produced. The exhaust gas occurred in the cement burning kiln 5 and the calcination furnace 4 during the production of the cement clinker goes through the preheater 3 from the lower side to the upper side, passes through the exhaust pipe 9 to be introduced into the material mill/drier 2 for drying the cement material, then is discharged from the chimney 11 via the dust collector 10.

In this process of producing cement, a part of the exhaust gas occurred when the cement is burned is collected to the exhaust gas collection line 311 of the methanation device 31 between the dust collector 10 and the chimney 11 of the exhaust gas treatment line 12. Next, the harmful component removal unit 312 removes the acidic component and the harmful component from the exhaust gas (harmful component removal step). In the harmful component removal unit 312, nitrogen oxides (NOx), sulfur oxides (SOx), halogen, H₂O, dusts and the like are removed. Then, CO₂ is taken out from the exhaust gas by the CO₂ separation/recover unit 313 and separated/recovered. At this time, the exhaust gas from which CO₂ is removed is discharged outside.

Next, the compression unit 314 compresses the recovered CO₂ to be 0.1 MPa or more by applying a pressure of 0.5 to 1.0 MPa, and then moisture included in CO₂ is removed by the dehumidification unit 315. Then, by the hydrogen mixing unit 316, hydrogen is supplied to the dehumidified CO₂ and mixed with it, then pressurized. Then, by the methane production unit 317, methane is generated from CO₂ in which hydrogen is mixed.

The methane generated in this manner is stored in the tank 322 of the methane supply device 32. The methane stored in the tank 322 is supplied to the cement burning kiln 5 and the calcination furnace 4 via the methane supply line 323. In the cement burning kiln 5, fossil fuel such as petroleum, coal of the like is supplied from the fuel supply line 15, however, some of the fossil fuel can be substituted with methane by supplying methane, and the fossil fuel can be reduced. Similarly, in the calcination furnace 4, some of the fuel such as coal is substituted with methane, so that fossil fuel can be reduced.

In the present embodiment, before separation and recover of the exhaust gas from the cement production facility 50, by removing the acidic components such as SO_{X}, NO_{X}, halogen and the like, and the harmful components such as H₂O, dusts and the like contained in CO₂ of the cement production exhaust gas, the deterioration of the absorption ability of the CO₂ absorption material can be suppressed. Accordingly, CO₂ can be efficiently recovered from the cement production exhaust gas. The CO₂ absorption material also can be maintained to have stable performance for a long time. By converting CO₂ appropriately treated to methane, CO₂ discharged from the cement production facility 50 can be reduced; and by using the methane as the alternative fuel for the cement burning kiln 5 and the calcination furnace 4, the methane can be effectively utilized. Notably, since methane substitutes for the fossil fuel such as coal and petroleum being the major cause of the global warming, the usage of the fossil fuel is decreased to reduce CO₂ derived from energy, and the reduction effect of the greenhouse gas can be improved.

The present invention is not limited to the above-described embodiments and various modifications may be made without departing from the scope of the present invention.

For example, although the generated methane is supplied to both the cement burning kiln 5 and the calcination furnace 4, it is possible to supply to either one of them.

Moreover, although the methane is generated using the exhaust gas from both the cement burning kiln 5 and the calcination furnace 4, it is possible to apply to a cement production facility having no calcination furnace; in this case, methane is generated from exhaust gas from a cement burning kiln.

### Industrial Applicability

Acidic components and harmful components is appropriately removed from CO₂ in exhaust gas of a cement production facility and deterioration of absorption ability of a CO₂ absorption material can be suppressed.

### Reference Signs List

- 1: Raw material storehouse
- 2: Material mill/dryer
- 3: Preheater
- 4: Calcination furnace
- 5: Cement burning kiln
- 5a: Kiln tail part
- 5b: Kiln front part
- 6: Cooler
- 8: Burner
- 9: Exhaust pipe
- 10: Dust collector
- 11: Chimney
- 12: Exhaust gas treatment line
- 13: Cyclone
- 15: Fuel supply line
- 22: Material supply pipe
- 25: Riser duct
- 30: Exhaust gas treatment facility
- 31: Methanation device
- 310: CO₂ separation/recover device
- 311: Exhaust gas collection line
- 312: Harmful component removal device
- 313: CO₂ separation/recover unit
- 314: Compression unit
- 315: Dehumidification unit
- 316: Hydrogen mixing unit
- 317: Methane production unit
- 32: Methane supply device
- 321: Pump
- 322: Tank
- 323: Methane supply line
- 41: Burner
- 42: Fuel supply line
- 50: Cement production facility
- 100: CO₂ utilizing system

## Claims

1. A CO₂ separation and recover method in cement production exhaust gas, comprising
a step of harmful component removal that removes an acidic component and a harmful component from exhaust gas discharged from a cement production facility; and
a step of CO₂ separation and recover that separates and recovers CO₂ by bringing the exhaust gas from which the acidic component and the harmful component are removed into contact with a CO₂ absorption material.

2. A CO₂ separation and recover device in cement production exhaust gas, comprising a cement production facility that is provided with
a harmful component removal unit that removes an acidic component and a harmful component from exhaust gas from the cement production facility, and
a CO₂ separation and recover unit that separates and recovers CO₂ by bringing the exhaust from which the acidic component and the harmful component are removed into contact with a CO₂ absorption material.
